# EUROPÄISCHE PATENTSCHRIFT

(11) **EP 3 323 366 B1**
(45) Veröffentlichungstag und Bekanntmachung des Hinweises auf die Patenterteilung: **30.09.2020**
(21) Anmeldenummer: 16199575.8
(22) Anmeldetag: 18.11.2016
(51) Int. Cl.: A61B 18/02

(54) **KRYOSONDE UND VERFAHREN ZUR HERSTELLUNG EINER SOLCHEN**
CRYOPROBE AND METHOD FOR PRODUCING SAME
CRYOSONDE ET SON PROCÉDÉ DE FABRICATION

(43) Veröffentlichungstag der Anmeldung: 23.05.2018
(73) Patentinhaber: Erbe Elektromedizin GmbH, 72072 Tübingen (DE)
(72) Erfinder: FISCHER, Klaus, 72202 Nagold (DE); KRONENTHALER, Jörg, 72145 Hirrlingen (DE); BRODBECK, Achim, 72555 Metzingen (DE); ADLER, Marcus, 72070 Tübingen (DE)
(74) Vertreter: Rüger Abel Patentanwälte PartGmbB

(56) Entgegenhaltungen:
- DE-A1-102008 026 635
- US-A1- 2009 287 202
- US-A1- 2012 253 336
- US-A1- 2014 378 970
- US-B1- 6 241 722

## Beschreibung

Die Erfindung betrifft ein Verfahren zur Herstellung einer Kryosonde sowie eine Kryosonde.

Kryosonden dienen in der Medizin zur Einwirkung auf biologisches Gewebe mittels Kälte. Eine solche Kryosonde ist beispielsweise der DE 10 2009 018 291 A1 zu entnehmen. Diese Kryosonde umfasst einen flexiblen Schlauch, an dessen distalem Ende ein für die spezifische Anwendung speziell geformter Kopf aus Metall vorgesehen ist, der intern über ein Kältemittel gekühlt werden kann. Damit kann erreicht werden, dass biologisches Gewebe an dem Kopf anfriert und zum Beispiel zwecks Biopsie von dem umliegenden Gewebe getrennt und entnommen werden kann.

Aus der US 6 241 722 B1 ist eine Kryosonde bekannt, die an ihrem distalen Ende eine Endkappe aufweist. Die Endkappe ist an einem Schlauch gehalten, in dessen Lumen eine Kapillare mit einer Düse angeordnet ist. Zur Funktionsbeeinflussung kann die Kapillare verschiedene Axialpositionen einnehmen.

Die Verbindung zwischen dem distal vorgesehenen Kopf und dem flexiblen Schlauch muss fluiddicht und zugfest sein. Zudem werden häufig Kryosonden mit sehr geringem Durchmesser gewünscht, um mit der Sonde auch in enge Lumen und Gefäße eines Patienten vordringen zu können.

Darüber hinaus wird häufig gewünscht, Kryosonden herstellerseitig in steriler Form bereitzustellen, so dass die Kryosonde ohne zusätzliche Sterilisationsbehandlung am Patienten Anwendung finden kann. Das Bestreben geht dahin, solche Instrumente kostengünstig als Einwegprodukte bereitzustellen.

Davon ausgehend ist es Aufgabe der Erfindung, ein Verfahren zur Bereitstellung von Kryosonden anzugeben, mit dem sich diese einfach und prozesssicher herstellen lassen. Weiter ist Aufgabe der Erfindung die Bereitstellung einer Kryosonde, die mit dem erfindungsgemäßen Verfahren herstellbar ist und wenigstens einige der sonstigen oben genannten Forderungen erfüllt.

Der auf das Herstellungsverfahren gerichtete Teil der genannten Aufgabe wird durch ein Verfahren nach Anspruch 1 gelöst:

Erfindungsgemäß sieht das Verfahren vor, dass zur Herstellung einer Kryosonde von einer mindestens zwei Kanäle aufweisenden Schlauchanordnung ausgegangen wird, wobei in einen der Kanäle eine Düse in Gestalt eines gesonderten, vorzugsweise vorgefertigten Bauteils eingefügt und das Schlauchende außen derart mit einer Hülse versehen wird, dass die Hülse das Schlauchende aufnimmt und distal über das Schlauchende, insbesondere über dessen stirnseitige, vorzugsweise ebene Stirnfläche übersteht. Danach wird die Hülse in einem Umformvorgang so nach innen verformt, dass sie mittels Pressverbindung auf dem Schlauchende fixiert ist. Alternativ kann die Hülse schon vor der Montage einen Innendurchmesser aufweisen, der geringer ist als der Außendurchmesser des Schlauchendes, das in diese Hülse dann axial eingepresst wird. In beiden Fällen weist die Hülse spätestens in fertig montiertem Zustand eine lichte innere Weite auf, die geringer ist, als der Außendurchmesser der Schlauchanordnung. Dadurch wird ein Presssitz der Hülse auf dem Schlauchende sichergestellt.

Die Hülse kann prinzipiell an ihrem distalen Ende geschlossen sein oder nachträglich geschlossen werden. Dazu kann eine Endkappe dienen, die zum distalen Verschluss der Hülse, mittels einer ringförmigen Dichtverbindung, z.B. einer Schweißnaht, abdichtend mit dieser verbunden wird. Die Ausbildung der Scheißnaht erfolgt vorzugsweise nach der Anbringung der Hülse auf dem Schlauchende. Alternativ kann die Endkappe auch vor dem Anbringen der Hülse nach einem der vorgenannten Verfahren mit dieser beispielsweise mittels ringförmiger Schweißnaht verbunden werden. Es ist auch möglich, die Hülse und die Endkappe einteilig, d.h. nahtlos einstückig aus ein und demselben Material auszubilden.

Alternativ zu der Metallcrimphülse können auch eine Sondenspitze z.B. aus wärmeleitendem Kunststoff und eine Kunststoffumspritzung verwendet werden kann. Dichtheit und Druckfestigkeit werden dann z.B. durch eine Ultraschallschweißung erreicht.

Die Herstellung der Kryosonde in dem genannten Verfahren mittels beidseitig offener Hülse wird bevorzugt. Die Hülse kann dann von einem Montagedorn aufgenommen werden, der eine Anlagefläche für die Hülse aufweist, an der die Hülse eine stirnseitige feste Anlage findet und die zur axialen Positionierung der Hülse bei der Montage auf dem Schlauchende dient. Der Montagedorn kann innerhalb dieser ringförmigen Anlagefläche einen Vorsprung aufweisen, der zur stirnseitigen Anlage des Schlauchendes dient, so dass die gewünschte Distanzierung der Endfläche der Hülse zu der Stirnfläche des Schlauchendes sichergestellt wird. Auf diese Weise kann in einem einfachen Fügevorgang sichergestellt werden, dass die Hülse bezüglich des Schlauchendes einen festen gewünschten Überstand aufweist.

Ebenso kann das Einschieben der Düse in das Schlauchende in diesem Arbeitsgang und unter Zuhilfenahme des Montagedorns erfolgen. Eine von dem Dorn aufgenommen oder zuvor mit einem Ende in dem Schlauchende platzierte Düse kann während des Fügevorgangs der Hülse und des Schlauchs von einer Düsenanlagefläche des Aufnahmedorns in das Schlauchende eingeschoben werden, so dass die Düse über ein gewünschtes Maß axial präzise über das Schlauchende hinausragt, d.h. insbesondere einen präzisen axialen Überstand über die Stirnfläche des Schlauchendes aufweist. Durch diesen Fügevorgang mittels des Montagedorns, der drei gesonderte Anlageflächen (für Hülse, Schlauchende und Düse) aufweist, wird erreicht, dass die Hülse, die Düse sowie die Stirnfläche des Schlauchendes präzise in der gewünschten gegenseitigen Maßbeziehung zueinander stehen. Nach dem Schließen der Hülse mittels einer Kappe werden dadurch in dem so geschaffenen Expansionsraum für das Kryofluid definierte Strömungsverhältnisse geschaffen, so dass auf einfache Weise die korrekte thermische Funktion der Kryosonde sichergestellt ist. Insbesondere kann sichergestellt werden, dass sich die Kryosonde gleichmäßig oder mit einer gewünschten Kälteverteilung abkühlt und somit in der Anwendung später den gewünschten chirurgischen Effekt erbringt.

Die derart herzustellende Kryosonde kann, falls gewünscht, ohne Zuhilfenahme von Klebstoffen bereitgestellt werden. Die Abdichtung zwischen dem Expansionsraum und dem Schlauch erfolgt durch das Kunststoffmaterial des Schlauchs selbst, der insoweit als Dichtung wirkt. Die Abdichtung zwischen der Kappe und der Hülse erfolgt beispielsweise durch die Schweißnaht. Die Abdichtung zwischen der Düse und der Innenwand des Kanals des Schlauchs wird durch das Schlauchmaterial selbst erbracht, das auch insoweit als Dichtung wirkt.

Das Fertigungsverfahren kommt, falls gewünscht, bei der Herstellung ohne flüssige Dicht- und Klebstoffe aus und ist tauglich zur Anwendung im Reinraum. Dies erleichtert die Herstellung als Sterilprodukt und reduziert den Aufwand dafür.

Die Schlauchanordnung der Kryosonde kann durch einen einzigen zwei oder mehrere Kanäle aufweisenden Schlauch ausgebildet sein. Es ist jedoch auch möglich, zwei oder mehrere Schläuche vorzusehen, die miteinander verbunden sind. Dazu sind sie beispielsweise an dem Schlauchende in einem Kunststoffkörper gefasst, der zur Aufnahme der Hülse dient. Die übrigen vor- oder nachstehend erläuterten Ausführungsvarianten sind bei einer solchen Schlauchanordnung ebenso gegeben wie bei einem einteiligen Schlauch der zwei oder mehrere Kanäle aufweist.

Die Düse ist ein separates Bauteil und kann insbesondere an ihrer ansonsten zylindrischen Außenumfangsfläche mit einer Verankerungsstruktur zur Verankerung in dem Schlauchende versehen sein. Die Verankerungsstruktur kann beispielsweise in lokalen oder auch sich um den Umfang herum erstreckenden, beispielsweisen ringförmigen Rippen gebildet sein, die beispielsweise einen Dreiecksquerschnitt aufweisen. Die Verankerungsstruktur kann auch durch Mikrovertiefungen oder Erhebungen, d.h. eine an der gesamten Außenumfangsoberfläche oder in Zonen derselben vorgesehenen Rauheit oder in einer Rändelung bestehen.

Das Schlauchende kann insbesondere in dem Bereich, in dem die Hülse nach innen verformt worden ist, d.h. in der Umformzone, in der sie eine radial nach innen gerichtete Kraft auf das Schlauchende ausübt, mit einer Stützstruktur versehen sein. Diese Stützstruktur kann durch die Düse selbst gebildet sein. Die Düse wird dadurch und, falls vorhanden, durch ihre Verankerungsstruktur in dem Kanal fest gehalten. Durch das radial nach innen gerichtete Verformen der Hülse wird a) die Hülse mit der später anzubringenden Endkappe axial unverschiebbar auf dem Schlauch und außerdem b) die Düse in dem Kanal des Schlauchs axial unverschiebbar gesichert.

Außerdem können in den freien, d.h. die Düse nicht aufnehmenden Kanälen Stützstrukturen angeordnet sein. Als Stützstruktur eignen sich beispielsweise Metallrohrabschnitte, Drahtgebilde, bspw. nach Art einer Schraubenfeder, oder auch eine Kunststoffauskleidung des betreffenden Kanals mit einem Kunststoff, dessen Steifigkeit höher ist als die Steifigkeit des übrigen Schlauchmaterials. Die Stützstruktur kann sich, insbesondere im Falle der steifen Kunststoffauskleidung, entlang der gesamten Länge des Schlauchs erstrecken. Ein solcher ein- oder mehrkanaliger Schlauch kann zum Beispiel als Co-Extrudat bereitgestellt werden.

Die radial nach innen gerichtete Verformung der Hülse kann durch ein Umformwerkzeuge mit zwei oder mehreren um den Umfang der Hülse herum angeordneten Klemmbacken erfolgen, die die Hülse in einem Pressvorgang so deformieren, dass ihr Innendurchmesser abnimmt und dass das Schlauchende festgeklemmt wird. Die Verformung kann auch durch Rollieren, z.B. mittels einer oder mehrerer entlang des Umfangs der Hülse umlaufender Rollen vorgenommen werden. Alternativ kann die Verformung berührungslos mittels eines Magnetumformverfahrens erfolgen. Dazu kann die Hülse in einer Magnetspule platziert werden, die mit einem Stromimpuls beaufschlagt wird, welcher Wirbelströme in der Hülse induziert. Der Wirbelstrom wechselwirkt mit dem Spulenstrom durch Lorenzkraft und verformt die Hülse wenigstens in einer ringförmigen Zone radial nach innen.

Alternativ kann die Hülse aus einem Formgedächtnismaterial, insbesondere Formgedächtnismetall, z.B. einer Nickel-Titan-Legierung (Nitinol), gefertigt sein. Die Hülse wird dann z.B. in einem kalt geweiteten Zustand mit einem Innendurchmesser bereitgestellt, der größer ist, als der Außendurchmesser des Schlauches, wobei sie bei Erwärmung in ihre Ursprungsform zurückfindet, in der der Innendurchmesser geringer ist, als der Außendurchmesser des Schlauches.

Weitere Einzelheiten vorteilhafter Ausführungsformen sind Gegenstand der Beschreibung der Zeichnung oder der Ansprüche. Es zeigen:
Figur 1 die erfindungsgemäße Kryosonde, in Übersichtsdarstellung,
Figur 2 die Kryosonde nach Figur 1, in abschnittsweiser längsgeschnittener Darstellung,
Figur 3 ein Prozessschritt des Herstellungsprozesses zur Bereitstellung der Kryosonde nach Figur1 und 2, unter Nutzung eines Montagedorns, in Explosionsdarstellung,
Figur 4 eine alternative Ausführungsform eines Montagedorns zur Durchführung des Verfahrens,
Figur 5 die Kryosonde nach Figur 2 während der Herstellung mit einem Montagedorn gemäß Figur 3 und einem Schlauch mit inhärenter innerer Abstützung,
Figur 6 den Montagedorn nach Figur 4, in Seitenansicht,
Figur 7 und 8 Schlauchanordnungen zur Bereitstellung der Kryosonde, in verschiedenen Ausführungsformen und in perspektivischer Darstellung,
Figur 9 und 10 Düsen für die Kryosonde in verschiedenen Ausführungsformen, jeweils in längsgeschnittener Darstellung und
Figur 11 die Kryosonde ohne Endkappe, in Stirnansicht zur Veranschaulichung des Befestigungsvorgangs der Hülse.

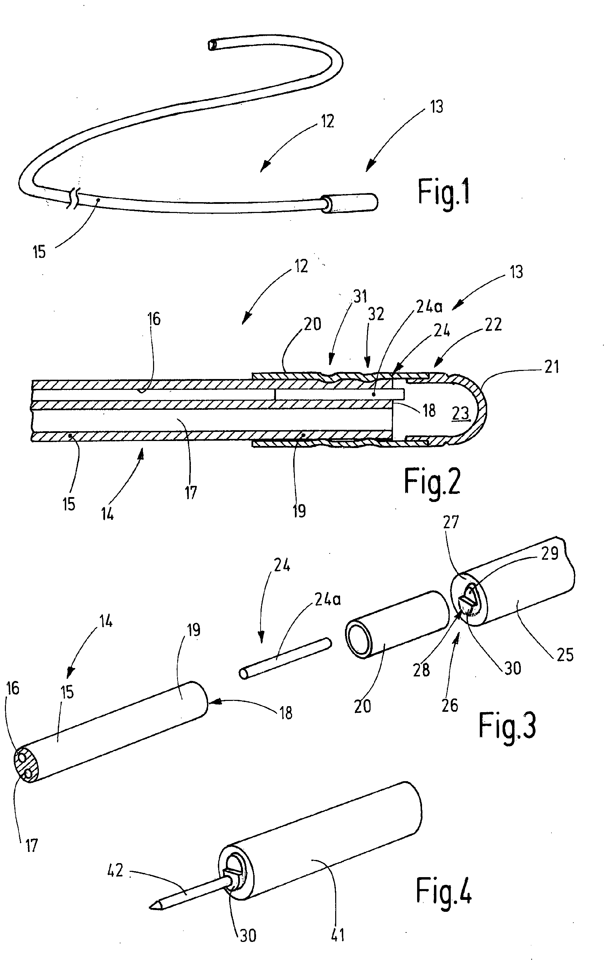

In Figur 1 ist eine Kryosonde 12 veranschaulicht, die zum Beispiel zur Kryobehandlung von biologischem Gewebe dienen kann. Zum Beispiel kann die Kryosonde 12 in einem Bronchoskop zur Entnahme einer Gewebeprobe dienen. Die Kryosonde 12 wird dazu zum Beispiel mittels eines flexiblen Bronchoskop in die Lunge beispielsweise bis in die Pleura eingeführt, wo dann der Kopf 13 mit biologischem Gewebe in Kontakt gebracht wird. Mittels eines expandierenden oder verdampfenden, d.h. gasförmigen oder flüssigen Kryofluids, wie z.B. N₂ oder CO₂, wird zumindest ein Abschnitt des Kopfes 13 dann soweit abgekühlt, dass anliegendes biologisches Gewebe gefriert, und, falls Gewebeentnahme gewünscht ist, an dem Kopf 13 anhaftet und mit diesem zusammen aus der Pleura entnommen werden kann.

Der Aufbau der Kryosonde 12 ist beispielhaft in Figur 2 veranschaulicht. Zu der Gewebesonde 12 gehört eine Schlauchanordnung 14, die im vorliegenden Ausführungsbeispiel durch einen flexiblen Kunststoffschlauch 15 gebildet wird, der einen ersten Kanal 16 sowie einen zweiten Kanal 17 aufweist. Beide Kanäle 16, 17 können unterschiedliche Durchmesser aufweisen. Vorzugsweise ist der Querschnitt des zweiten Kanals 17 1,1 bis 2,5 mal größer als der Querschnitt des ersten Kanals 16. Beide Kanäle 16, 17 erstrecken sich vorzugsweise parallel zueinander und im Abstand nebeneinander durch die gesamte Länge des Kunststoffschlauchs 15 und münden beide an der distalen, vorzugsweise ebenen Stirnfläche 18 desselben.

Der Kunststoffschlauch 15 weist ein Schlauchende 19 auf, das den Kopf 13 der Kryosonde 12 trägt. Der Kopf 13 umfasst eine Hülse 20, die auf dem Schlauchende 19 gehalten ist und die Stirnfläche 18 überragt. Die Hülse 20 trägt eine Endkappe 21, die mit der Hülse fluiddicht verbunden ist. Vorzugsweise ist die Endkappe 21 dazu an dem distalen Ende der Hülse 20 mit dieser zum Beispiel bei einer ringförmigen Laserschweißnaht oder anderweitige Schweißnaht verschweißt. Die Endkappe 21 begrenzt somit distal einen Expansionsraum 23 für das Kryofluid, das über den ersten Kanal 16 herangeführt und über eine Düse 24 in den Expansionsraum 23 eingeführt wird. Die Düse ist ein Bauteil, das aus Metall, Keramik oder auch einem Kunststoff bestehen kann, wobei der Kunststoff vorzugsweise ein von dem Material des Kunststoffschlauchs verschiedener Kunststoff ist.

Die Düse 24 ist mit ihrem Düsenschaft 24a in dem sich an die distale Stirnfläche 18 anschließenden Endabschnitt des ersten Kanals 16 gehalten, z.B. festgeklemmt. Die Düse 24 kann endseitig mit der distalen Stirnfläche 18 abschließen oder, wie es bevorzugt wird und in Figur 2 dargestellt ist, etwas aus dem Kanal 16 heraus in den Expansionsraum 23 hinein ragen. Die Axialposition der Düse beeinflusst dabei die Strömungsverhältnisse in der Expansionskammer 23 und ist somit für die korrekte Funktion wesentlich.

Vorzugsweise weist die Düse 24 eine im Wesentlichen runde Düsenöffnung auf, die in der Düse 24 zentrisch und somit zentrisch zu dem Kanal angeordnet ist, in dem die Düse gehalten ist. Dies vereinfacht die Herstellung, weil keine Ausrichtung der Düse nötig ist, bevor sie in den Kanal 16 eingeschoben wird. Allerding ist auch eine asymmetrische Anordnung möglich, was der Kälteverteilung zugute kommen kann.

Die Herstellung der insoweit beschriebenen Kryosonde 12 ist zumindest teilweise in Figur 3 veranschaulicht. Es wird dazu der Kunststoffschlauch 15 zunächst mit der Düse 24 versehen, die zumindest soweit in den Kanal 16 eingeführt wird, dass sie darin vorläufig gehalten ist. Im Weiteren wird die Hülse 20 auf einen Montagedorn 25 aufgesetzt, der dazu eine Hülsenaufnahme 26 aufweist. Diese umfasst eine ringförmige, vorzugsweise ebene Druckfläche 27, die einen Vorsprung 28 umgibt. Dieser Vorsprung 28 weist eine zum Beispiel zylindrische Außenumfangsfläche auf, deren Außendurchmesser dem Innendurchmesser der Hülse 20 entspricht, so dass die Hülse 20 auf den Vorsprung 28 aufgesteckt werden kann und dann auf diesem gehalten ist. Der Vorsprung 28 und die Druckfläche 27 bilden zusammen einen Sitz für die Hülse 20.

Der Vorsprung 28 ist an seiner Stirnseite vorzugsweise gestuft ausgebildet. Er weist eine erste Anlagefläche 29 für die Düse 24 und eine zweite Anlagefläche 30 auf, die bei der Montage mit der distalen Stirnfläche 18 des Kunststoffschlauchs 15 im Bereich des zweiten Kanals 17 in Berührung kommen soll.

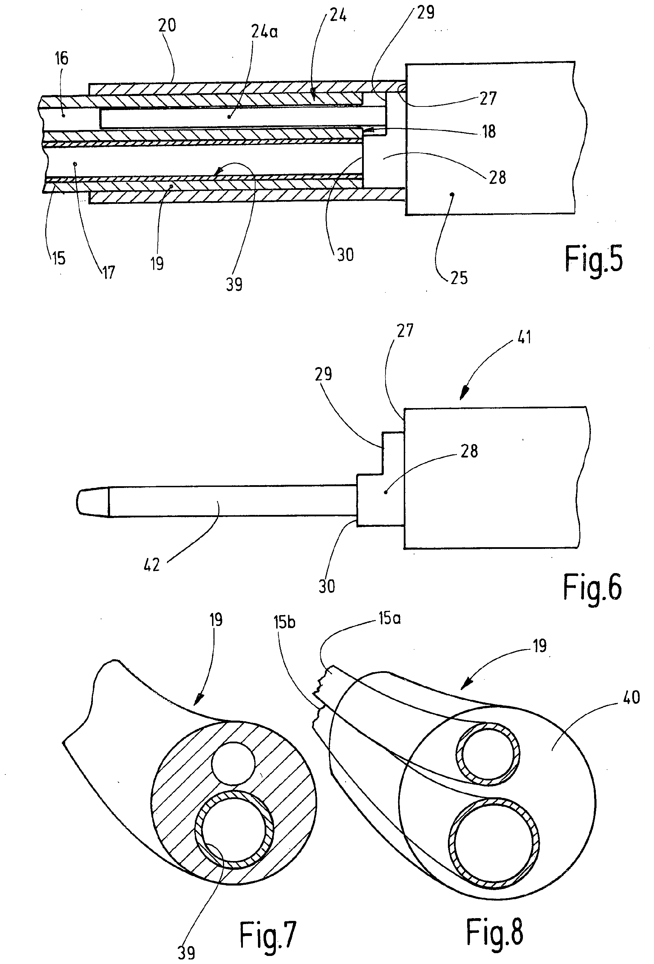

Die Montage der Hülse 20 und der Düse 24 an dem Schlauchende 19 des Kunststoffschlauchs 15 ergibt sich aus Figur 5. Der Kunststoffschlauch 15, in den die Düse 24 mit ihrem Düsenschaft 24a teilweise eingesteckt ist, und der mit der Hülse 20 versehene Montagedorn 25 werden axial so aufeinander zu bewegt, dass zunächst das Schlauchende 19 in die Hülse 20 einfährt und die erste Anlagefläche 29 mit der Endfläche der Düse 24 in Berührung kommt. Die Axialbewegung wird dann so lange fortgesetzt, bis die zweite Anlagefläche 30 des Vorsprungs 28 mit der distalen Stirnfläche 18 des Kunststoffschlauchs 15 in Berührung kommt. In diesem Zustand weisen die Hülse 20 und die Düse 24 in Bezug auf die die distale Stirnfläche 18 wohldefinierte Axialpositionen auf, womit eine wesentliche Grundlage für die spätere korrekte Funktion der Kryosonde 12 geschaffen ist.

Der insoweit beschriebene Fügevorgang kann sowohl bei einer ersten Ausführungsform, bei der die Hülse 20 einen geringeren Durchmesser aufweist als der Außendurchmesser des Kunststoffschlauchs 15, als auch bei einer zweiten Ausführungsform Anwendung finden, bei der der Innendurchmesser der Hülse 20 wenigstens so groß ist wie der Außendurchmesser des Kunststoffschlauchs 15.

Bei der erstgenannten Ausführungsform kann die Hülse 20 an ihrem proximalen Ende eine nicht weiter veranschaulichte trichterartige Einführschräge aufweisen. Alternativ oder zusätzlich kann die distale Stirnfläche 18 des Kunststoffschlauchs 15 an ihrem radial äußeren Rand in eine Kegelfläche übergehen, die eine schlauchseitige Einführschräge bildet. Es ist möglich, dadurch die Hülse 20 im Presssitz auf dem Schlauchende 19 und die Düse 24 im Presssitz in dem Schlauchende 19 zu sichern. Bei der zweiten oben schon genannten Ausführungsform wird die Hülse 20 nach ihrer Aufbringung auf das Schlauchende 19 wenigstens stellenweise nachträglich radial nach innen verformt und somit verengt. Figur 2 veranschaulicht eine solche Hülse 20 mit zwei axial voneinander beabstandeten, jeweils ringförmigen, über den gesamten Umfang der Hülse 20 umlaufenden Presszonen 31, 32, die durch plastische Verformung der Hülse erreicht worden sind. Die plastische Verformung kann durch zwei oder mehrere, die Hülse 20 zwischen einander aufnehmende und während des Pressvorgangs radial nach innen bewegende Klemmbacken, durch ein Rollierwerkzeug mit einer den Umfang der Presshülse 20 ein oder mehrmals umrundenden Rolle oder mit mehreren solchen Rollen gebildet sein. Außerdem kann die Hülse durch Magnetumformung durch ein gepulstes Magnetfeld zonenweise oder im Ganzen verengt, d.h. radial nach innen umgeformt werden. Dabei ist prinzipbedingt kein mechanischer Kontakt zum Werkstück erforderlich, so dass Oberflächenverunreinigungen der Hülse ausgeschlossen werden können. Das Verfahren lässt sich unter Reinraumbedingungen anwenden.

Ergänzend oder alternativ ist es bei allen vorgenannten Ausführungsformen möglich, die Hülse 20 mit einem geeigneten Verbindungsmittel beispielsweise einem Klebstoff auf dem Schlauchende 19 zu sichern. Es kann sich dabei um Zwei-Komponentenkleber (Polyurethankleber oder Epoxidharzkleber), hochelastisches Cyanacrylat, einen UV-aushärtbaren Kleber, einen aerob-aushärtenden Kleber, einem anaerob-aushärtenden Kleber oder einen lösungsmittelhaltigen Kleber handeln. Das Schlauchende 19 kann zur Haftungsverbesserung vorbehandelt sein. Beispielsweise kann dies durch Fluorierung, Ätzung, mechanisehe Behandlung, zum Beispiel Aufrauhung, Plasmaaktivierung oder mittels eines Primers erfolgen. Bevorzugt wird jedoch auf Klebemittel verzichtet.

Die Düse 24 (d.h. insbesondere der Düsenschaft 24a) ist erfindungsgemäß ebenfalls im Presssitz gehalten. Dazu kann die Düse 24 einen etwas größeren Außendurchmesser aufweisen als das erste Lumen 16, in dem sie gehalten ist. Unterstützend oder alternativ kann der Presssitz auch durch eine wenigstens zonenweise Verengung des Lumens 16 infolge der Pressung erfolgen, die von der Hülse 20 radial nach innen gerichtet auf das Schlauchende 19 ausgeübt wird.

Nach dem Anbringen der Düse 24 und der Hülse 20 an dem Schlauchende 19 wird die Endkappe 21 angebracht. Diese kann zur Erleichterung der Positionierung einen ringförmigen oder mehrere z.B. drei fingerförmige oder nach Art von Laschen ausgebildete Fortsätze aufweisen, die in den über die distale Stirnfläche 18 überstehenden Teil der Hülse 20 fassen. Der Fortsatz kann auch als Ringfortsatz mit einer oder mehreren Unterbrechungen ausgebildet sein. Die zunächst aufgesteckte Endkappe 21 kann dann mit einem geeigneten Füge- oder Schweißverfahren, beispielsweise Laserschweißen fluiddicht mit der Hülse 20 verbunden werden.

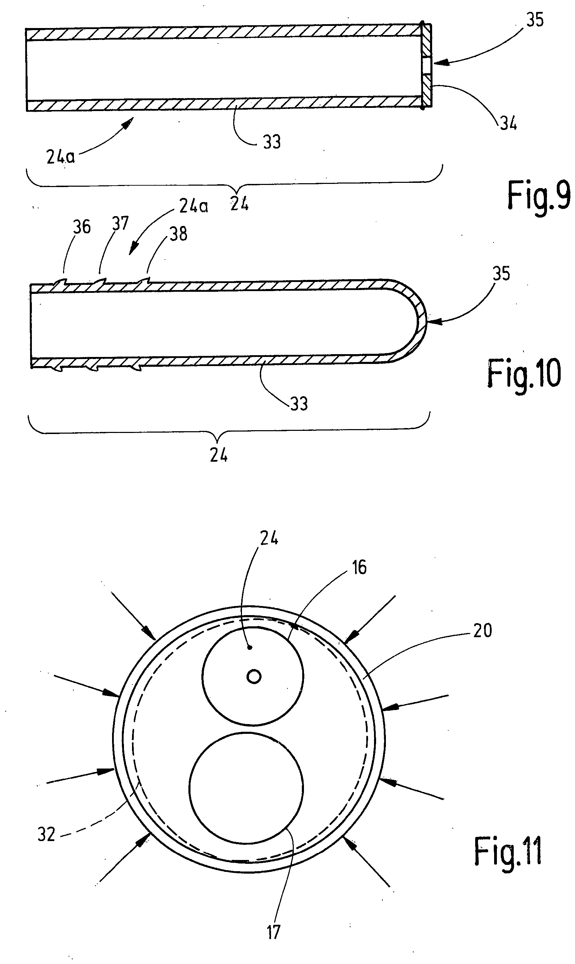

Figur 11 veranschaulicht die Ausbildung der Presszone 32 in gestrichelter Form. Wie ersichtlich, kann die Tiefe der Presszone 32 um den Umfang der Hülse 20 herum variieren. Zum Beispiel kann die Tiefe der Presszone 32 in unmittelbarer Nachbarschaft zu den Kanälen 16, 17 gegenüber der sonstigen Tiefe der Presszone 32 verringert sein, um eine Deformation insbesondere eine Verengung der Kanäle 16, 17 in Grenzen zu halten. Es ist auch möglich, die Tiefe der Presszone 32 lediglich im Bereich des zweiten Kanals 17 zu reduzieren, um einen Kollaps des zweiten Kanals 17 zu verhindern, während die Presswirkung auf den ersten Kanal 16 die Sicherung der Düse 24 in dem ersten Kanal 16 fördert.

Weiter ist es möglich, die Düse 24 in den Kanal 16 axial zusätzlich zu sichern. Figur 9 veranschaulicht eine Düse 24, die einen glattwandigen Rohrabschnitt 33 aufweist, der an einem Ende durch ein Düsenplättchen 34 verschlossen ist, das mindestens eine Düsenöffnung 36 aufweist. Das Düsenplättchen 34 kann mit dem Rohrabschnitt 33 verschweißt sein, beispielsweise durch Laserschweißen. Es ist aber auch möglich, anstelle des Düsenplättchens 34 das distale Ende des Rohrabschnitts 33 in einem Umformvorgang zu verengen, um so eine verengte Düsenöffnung 35 auszubilden. Die Verengung kann zu dem Rohrabschnitt 33 koaxial oder auch asymmetrisch, z.B. außermittig oder mit schräg zu der Axialrichtung des Rohrabschnitts 33 orientierter Achse erfolgen.

In beiden Fällen können die Strukturen zur axialen Sicherung der Düse in dem Lumen 16 durch Vorsprünge, zum Beispiel ringförmige Zahnrippen 36, 37, 38, durch eine oder mehrere nach Art eines Gewindes ausgebildete Rippen, Noppen, unregelmäßige Strukturen wie Rauheiten oder durch Rändelung ausgebildet sein.

Der erste Kanal 16 dient dem Fluidzustrom, d.h. der Speisung der Düse 24 mit flüssigem oder gasförmigem Kryofluid. Der zweite Kanal 17 dient zur Abfuhr des Kryofluids aus dem Expansionsraum 23. Um ein Verengen des zweiten Kanals 17 insbesondere im Bereich der Hülse 20 zu mindern oder zu verhindern, ist es möglich, in dem Schlauchende 19 oder entlang des gesamten Kanals 17 eine Stützstruktur 39 vorzusehen. Eine solche ist in Figur 5 schematisch veranschaulicht. Die Stützstruktur 39 besteht dort aus einer Kunststoffauskleidung des zweiten Kanals 17, wie sie auch aus Figur 7 hervorgeht. Während der Kunststoffschlauch 15 generell aus Polyamiden, Polyolefinen, Pebax, Polyurethan, PEEK, PI, Verbundwerkstoffe oder anderen Kunststoffen bestehen kann, kann die Stützstruktur 39 aus einem vergleichsweisen steiferen Kunststoff oder einem Metallgeflecht bestehen. Beschränkt sich die Stützstruktur auf das Schlauchende 19 kann sie auch aus einem Metallrohr bestehen.

Die Schlauchanordnung 14 kann, wie in Figur 8 dargestellt, auch aus mehreren, zum Beispiel zwei Kunststoffschläuchen 15, 15b gebildet sein, die gleiche oder unterschiedliche Durchmesser aufweisen und im Bereich des Kopfs in einem Körper vorzugsweise einem Kunststoffkörper 40 eingebettet sind. Dieser bildet dann das Schlauchende 19, wobei ansonsten die vorige Beschreibung entsprechend gilt.

Die Herstellung, insbesondere das Fügen des Schlauchendes 19 mit der Düse 24 und der Hülse 20 erfolgt erfindungsgemäß mit einem Montagedorn 41 nach Figur 4 und 6. Bei diesem ist auf der zweiten Anlagefläche 30 ein Stützdorn 42 angeordnet, dessen Außendurchmesser im Wesentlichen mit dem Innendurchmesser des zweiten Kanals 17 übereinstimmt oder geringfügig kleiner ist als dieser. Bei der Montage fährt der Stützdorn 41 in den zweiten Kanal 17 ein und verbleibt insbesondere auch während der radial nach innen gerichteten Umformung der Hülse 20 in dem Kanal 17. Der Stützdorn 42 verhindert dabei, dass der Kanal 17 infolge der radial nach innen gerichteten Umformung der Hülse 20 kollabiert oder zu sehr verengt wird.

Die insoweit beschriebenen Herstellungsverfahren können nachfolgende weitere Abwandlung(en) erfahren:

Der Stützdorn 42 kann zur Aufnahme eines dünnwandigen Röhrchens dienen, das die stirnseitige Anlage an der zweiten Anlagefläche 30 findet und beim Fügevorgang in den zweiten Kanal 17 engeschoben wird, um dort einen Kollaps des zweiten Kanals 17 bei der radial nach innen gerichtete Deformation der Hülse 20 zu verhindern. Weiter ist es möglich, den Vorsprung 28 ohne Stufe auszubilden, wobei die Anlagefläche 29 in einer Vertiefung der Anlagefläche 30 ausgebildet ist. Die Vertiefung, deren Boden durch die Anlagefläche 29 gebildet wird, dient dann zur Aufnahme des distalen Endes der Düse 24, die dann während des Fügevorgangs in das erste Lumen 16 eingeschoben wird. Die Tiefe der Vertiefung bestimmt wiederum den Überstand der Düse 24 über die distale Stirnfläche 18 in fertig montiertem Zustand. Diese Ausführungsform kann sowohl an dem Montagedorn 25 nach Figur 3 wie auch an dem Montagedorn 41 nach Figur 4 und 6 verwirklicht werden. Die Vertiefung kann zur Aufnahme zylindrischer, rotationssymmetrischer Düsen rund sein. Sind die Düsen nicht rotationssymmetrisch, etwa weil die Düsenöffnung außermittig oder schräg strahlend ausgebildet ist, kann die Düse eine Verdrehsicherungsstruktur aufweisen und dazu z.B. eine Nase oder eine Ausnehmung aufweisen oder auf andere geeignete Weise unrund sein. Die Vertiefung ist dann entsprechend unrund ausgebildet.

Das erfindungsgemäße Verfahren zur Herstellung einer Kryosonde nutzt einen Montagedorn 25 zur Aufnahme einer Hülse 20, die einen Teil des Kopfs 13 der Kryosonde bilden soll und weist drei axial gegeneinander versetzte Anlageflächen 27, 29, 30 auf, die nach Montage der Hülse 20 und der Düse 24 an dem Schlauchende 19 die korrekte Axialpositionierung, insbesondere der Düse 24 und der Hülse 20 in Bezug auf die distale Endfläche 18 des Schlauchendes 19 sicherstellen. Damit ist die Position der Düse 24 in der nach Schließen der Hülse 20 erzeugten Expansionskammer 23 und somit die Funktion der Kryosonde sichergestellt.

**Bezugszeichen:**

| | |
|---|---|
| 12 | Kryosonde |
| 13 | Kopf der Kryosonde 12 |
| 14 | Schlauchanordnung |
| 15 | zweilumiger Kunststoffschlauch |
| 16 | erster Kanal der Schlauchanordnung 14 |
| 17 | zweiter Kanal der Schlauchanordnung 14 |
| 18 | distale Stirnseite des Kunststoffschlauchs 15 |
| 19 | Schlauchende des Kunststoffschlauchs 15 |
| 20 | Hülse |
| 21 | Endkappe |
| 22 | Laserschweißnaht |
| 23 | Expansionsraum |
| 24 | Düse |
| 24a | Düsenschaft |
| 25 | Montagedorn |
| 26 | Hülsenaufnahme |
| 27 | Druckfläche |
| 28 | Vorsprung / Sitz |
| 29 | erste Anlagefläche des Vorsprungs 28 |
| 30 | zweite Anlagefläche des Vorsprungs 28 |
| 31, 32 | Press zonen |
| 33 | glattwandiger Rohrabschnitt |
| 34 | Düsenplättchen |
| 35 | Düsenöffnung |
| 36, 37, 38 | Zahnrippen |
| 39 | Stützstruktur |
| 40 | Kunststoffkörper |
| 41 | Montagedorn |
| 42 | Stützdorn |

## Patentansprüche

1. Verfahren zur Herstellung einer Kryosonde (12), bei dem:
eine aus Kunststoffmaterial bestehende Schlauchanordnung (14) bereitgestellt wird, die wenigstens zwei Kanäle (16, 17) aufweist,
in einen der Kanäle (16) an einem Schlauchende (19) eine Düse (24) eingefügt wird,
das Schlauchende (19) mit einer (20) Hülse, deren Innendurchmesser wenigstens so groß ist, wie der Außendurchmesser der Schlauchanordnung (14), derart versehen wird, dass die Hülse (20) das Schlauchende (19) aufnimmt und distal über dieses übersteht, und die Hülse (20) danach radial nach innen verformt wird, um die Hülse (20) mittels Pressverbindung auf dem Schlauchende (19) zu fixieren oder das Schlauchende (19), das einen Außendurchmesser aufweist, der größer ist als der Innendurchmesser der Hülse (20), in diese Hülse (20) axial eingepresst wird, **dadurch gekennzeichnet,**
**dass** in demjenigen Kanal (17), in dem keine Düse platziert ist, während des Schritts der Verformung ein in das Ende des Kanals (17) eingeschobener Stützdorn (42) platziert ist.

2. Verfahren nach Anspruch 1, **dadurch gekennzeichnet, dass** die Hülse (20) nach ihrer Anbringung auf dem Schlauchende (19) distal verschlossen wird.

3. Verfahren nach Anspruch 2, **dadurch gekennzeichnet, dass** zum distalen Verschluss der Hülse (20) eine Endkappe (21) genutzt wird, die mit der Hülse (29) mittels einer ringförmigen dichten Verbindung, z.B. einer Schweißnaht (22), abdichtend verbunden wird.

4. Verfahren nach einem der vorstehenden Ansprüche, **dadurch gekennzeichnet, dass** zum Einschieben der aus Metall, Keramit oder Kunststoff bestehenden Düse (24) in den Kanal (16) und zum Aufschieben der Hülse (20) auf das Schlauchende (19) in einem Arbeitsgang ein Montagedorn (25, 41) verwendet wird, der einen Sitz (27, 28) für die Hülse (20) und eine Anlagefläche (29) für die Düse (24) aufweist, die einen axialen Abstand zueinander haben, der mit dem gewünschten axialen Abstand zwischen dem distalen Ende der Düse (24) und dem distalen Ende der Hülse (20) übereinstimmt.

5. Verfahren nach einem der der vorstehenden Ansprüche, **dadurch gekennzeichnet, dass** derjenige Kanal (17), in dem keine Düse platziert ist, während des Schritts der Verformung und nach demselben durch eine Stützstruktur (3) radial abgestützt wird, die den Kanal (17) umschließt.

6. Kryosonde (12):
mit einer Schlauchanordnung (14), die einen ersten Kanal (16) aufweist, in dessen distalen Ende eine Düse (24) mit einem Düsenschaft (24a) angeordnet ist, und die mindestens einen parallel und im Abstand zu dem ersten Kanal (16) angeordneten zweiten Kanal (17) aufweist, dessen Querschnitt größer ist als der Querschnitt der Düse (24) und/oder des ersten Kanals (16),mit einer Hülse (20), die auf einem distalen Schlauchende (19) der Schlauchanordnung (14) angeordnet ist und deren lichte innere Weite wenigstens in fertig montiertem Zustand geringer ist, als der Außendurchmesser der Schlauchanordnung (14), wodurch die Düse (24) in dem Kanal (16) im Pressitz gehalten ist, und
mit einer Endkappe (21), die an dem distalen Ende der Hülse (20) diese verschließend angeordnet ist.

7. Kryosonde nach Anspruch 6, **dadurch gekennzeichnet, dass** die Hülse (20) auf dem Schlauchende (19) im Presssitz gehalten ist.

8. Kryosonde nach Anspruch 6 oder 7, **dadurch gekennzeichnet, dass** die Hülse (20) und die Endkappe (21) durch eine dichte Verbindung, z.B. eine Ringschweißnaht (22) miteinander fluiddicht verbunden sind.

9. Kryosonde nach einem der Ansprüche 6 bis 8, **dadurch gekennzeichnet, dass** die Hülse (20) und die Endkappe (21) miteinander nahtlos einstückig ausgebildet sind.

10. Kryosonde nach einem der Ansprüche 6 bis 9, **dadurch gekennzeichnet, dass** die Düse (24) aus dem Schlauchende (19) herausragend platziert ist, wobei der Düsenschaft (24a) in dem Kanal (16) steckt.

11. Kryosonde nach einem der Ansprüche 6-10, **dadurch gekennzeichnet, dass** die Düse (24) mit einer Verankerungsstruktur (36, 37, 38) versehen ist.

12. Kryosonde nach einem der Ansprüche 6 bis 11, **dadurch gekennzeichnet, dass** die Düse (24) die Hülse (20) oder wenigstens eine Presszone (31, 32) derselben durchgreifend angeordnet ist.

13. Kryosonde nach einem der Ansprüche bis 12, **dadurch gekennzeichnet, dass** der zweite Kanal (17) mit einer Stützstruktur (39) versehen ist.

14. Kryosonde nach einem der Ansprüche 6 bis 13, **dadurch gekennzeichnet, dass** die Hülse (20) wenigstens in einer Presszone (31, 32) radial nach innen verformt ist, um das Schlauchende (19) zu komprimieren.

## Claims

1. Method for producing a cryoprobe (12) in which:
a hose arrangement (14) is provided which consists of a plastic material and has at least two channels (16, 17),
a nozzle (24) is inserted in one of the channels (16) at a hose end (19),
the hose end (19) is provided with a sleeve (20), the inner diameter of which is at least as large as the outer diameter of the hose arrangement (14), such that the sleeve (20) receives the hose end (19) and protrudes distally over this, and the sleeve (20) is then deformed radially inwardly in order to fix sleeve (20) to the hose end (19) by press fit, or the hose end (19) which has an outer diameter that is greater than the inner diameter of the sleeve (20) is pressed axially into said sleeve (20),
**characterised in that** during the step of deformation, a supporting mandrel (42) inserted in the end of the channel (17) is placed in the channel (17) in which no nozzle is placed.

2. Method according to claim 1, **characterised in that** after application to the hose end (19), the sleeve (20) is closed distally.

3. Method according to claim 2, **characterised in that** an end cap (21) is used for distal closure of the sleeve (20), wherein said end cap is tightly connected to the sleeve (20) by means of an annular sealed connection, e.g. a weld seam (22).

4. Method according to any of the preceding claims, **characterised in that** for inserting the nozzle (24) consisting of metal, ceramic or plastic into the channel (16), and for pushing the sleeve (20) onto the hose end (19) in one working step, a mounting mandrel (25, 41) is used which has a seat (27, 28) for the sleeve (20) and a contact face (29) for the nozzle (24) which are axially spaced from each other by a distance which corresponds to the desired axial distance between the distal end of the nozzle (24) and the distal end of the sleeve (20).

5. Method according to any of the preceding claims, **characterised in that** during the step of deformation and thereafter, the channel (17) in which no nozzle is placed is radially supported by a supporting structure (3) which surrounds the channel (17).

6. Cryoprobe (12),
with a hose arrangement (14) having a first channel (16), in the distal end of which a nozzle (24) with a nozzle shank (24a) is arranged, and having at least one second channel (17) which is arranged parallel to and spaced from the first channel (16) and the cross-section of which is greater than the cross-section of the nozzle (24) and/or of the first channel (16),
with a sleeve (20) which is arranged on a distal hose end (19) of the hose arrangement (14) and the clear inner width of which, at least in ready mounted state, is smaller than the outer diameter of the hose arrangement (14), whereby the nozzle (24) is held in the channel (16) by press fit, and
with an end cap (21) which is arranged on the distal end of the sleeve (20) so as to seal the latter.

7. Cryoprobe according to claim 6, **characterised in that** the sleeve (20) is held on the hose end (19) by press fit.

8. Cryoprobe according to claim 6 or 7, **characterised in that** the sleeve (20) and the end cap (21) are connected together fluid-tightly by a sealed connection, e.g. a ring weld seam (22).

9. Cryoprobe according to any of claims 6 to 8, **characterised in that** the sleeve (20) and the end cap (21) are formed integrally and seamlessly together.

10. Cryoprobe according to any of claims 6 to 9, **characterised in that** the nozzle (24) is placed protruding from the hose end (19), the nozzle shank (24a) being inserted in the channel (16).

11. Cryoprobe according to any of claims 6 to 10, **characterised in that** the nozzle (24) is provided with an anchoring structure (36, 37, 38).

12. Cryoprobe according to any of claims 6 to 11, **characterised in that** the nozzle (24) is arranged so as to extend through the sleeve (20) or at least a pressing zone (31, 32) thereof.

13. Cryoprobe according to any of claims 6 to 12, **characterised in that** the second channel (17) is provided with a supporting structure (39).

14. Cryoprobe according to any of claims 6 to 13, **characterised in that** the sleeve (20) is radially inwardly deformed, at least in a pressing zone (31, 32), in order to compress the hose end (19).

## Revendications

1. Procédé de fabrication d'une cryosonde (12), selon lequel :
un agencement de tuyau souple (14) est mis en place, qui est constitué d'une matière plastique et présente au moins deux conduites (16, 17),
une buse (24) est insérée dans l'une des conduites (16), à une extrémité de tuyau (19),
l'extrémité de tuyau (19) est munie d'une douille (20) dont le diamètre intérieur est au moins aussi grand que le diamètre extérieur de l'agencement de tuyau souple (14), de telle sorte que la douille (20) accueille l'extrémité de tuyau (19) et dépasse avec sa partie distale par rapport à celle-ci, et la douille (20) est ensuite radialement déformée vers l'intérieur afin de fixer la douille (20) par ajustement serré sur l'extrémité de tuyau (19), ou l'extrémité de tuyau (19), qui présente un diamètre extérieur supérieur au diamètre intérieur de la douille (20), est pressé axialement dans ladite douille (20),
**caractérisé en ce que**
pendant l'étape de déformation, un mandrin de contre-appui (42) est placé dans la conduite (17) dans laquelle il n'est pas prévu de buse et est glissé dans l'extrémité de la conduite (17).

2. Procédé selon la revendication 1, **caractérisé en ce que**, après son montage sur l'extrémité de tuyau (19), la douille (20) est fermée dans sa partie distale.

3. Procédé selon la revendication 2, **caractérisé en ce que** pour la fermeture distale de la douille (20), on utilise un capuchon d'extrémité (21) qui est relié de façon étanche à la douille (29) au moyen d'un assemblage annulaire étanche, par exemple un joint de soudure (22).

4. Procédé selon l'une des revendications précédentes, **caractérisé en ce que** pour insérer la buse (24), réalisée en métal, céramite ou matière plastique, dans la conduite (16) et pour rapporter la douille (20) sur l'extrémité de tuyau (19) au cours d'une opération de travail, on utilise un mandrin de montage (25, 41) présentant un siège (27, 28) pour la douille (20) et une surface d'appui (29) pour la buse (24), qui présentent un espacement axial l'un par rapport à l'autre qui correspond à l'espacement axial souhaité entre l'extrémité distale de la buse (24) et l'extrémité distale de la douille (20).

5. Procédé selon l'une des revendications précédentes, **caractérisé en ce que** pendant l'étape de déformation et après celle-ci, la conduite (17) ne contenant pas de buse est soutenue radialement par une structure de support (3) qui entoure la conduite (17).

6. Cryosonde (12) :
comprenant un agencement de tuyau souple (14) qui présente une première conduite (16), dans l'extrémité distale de laquelle est disposée une buse (24) dotée d'un fût de buse (24a), et qui présente au moins une deuxième conduite (17) qui est disposée parallèlement et à distance par rapport à la première conduite (16) et dont la section transversale est supérieure à la section transversale de la buse (24) et/ou de la première conduite (16), comprenant une douille (20) qui est disposée sur une extrémité de tuyau (19) distale de l'agencement de tuyau souple (14) et dont la largeur intérieure est inférieure, au moins à l'état monté final, au diamètre extérieur de l'agencement de tuyau souple (14), grâce à quoi la buse (24) est tenue par ajustement serré dans la conduite (16), et comprenant un capuchon d'extrémité (21) qui est disposé sur l'extrémité distale de la douille (20), en fermant celle-ci.

7. Cryosonde selon la revendication 6, **caractérisée en ce que** la douille (20) est tenue par ajustement serré sur l'extrémité de tuyau (19).

8. Cryosonde selon la revendication 6, **caractérisée en ce que** la douille (20) et le capuchon d'extrémité (21) sont reliés l'un à l'autre de manière étanche aux fluides par un assemblage étanche, par exemple un joint de soudure annulaire (22).

9. Cryosonde selon l'une des revendications 6 à 8, **caractérisée en ce que** la douille (20) et le capuchon d'extrémité (21) sont réalisés d'une seule pièce l'un avec l'autre, sans joint.

10. Cryosonde selon l'une des revendications 6 à 9, **caractérisée en ce que** la buse (24) est disposée de manière à dépasser de l'extrémité de tuyau (19), le fût de buse (24a) étant inséré dans la conduite (16).

11. Cryosonde selon l'une des revendications 6 à 10, **caractérisée en ce que** la buse (24) est dotée d'une structure d'ancrage (36, 37, 38).

12. Cryosonde selon l'une des revendications 6 à 11, **caractérisée en ce que** la buse (24) est disposée de manière à traverser la douille (20) ou au moins une zone d'ajustement serré (31, 32) de celle-ci.

13. Cryosonde selon l'une des revendications 6 à 12, **caractérisée en ce que** la deuxième conduite (17) est dotée d'une structure de support (39).

14. Cryosonde selon l'une des revendications 6 à 13, **caractérisée en ce que** la douille (20) est déformée radialement vers l'intérieur, au moins dans une zone d'ajustement serré (31, 32), aux fins de comprimer l'extrémité de tuyau (19).
